# EUROPÄISCHE PATENTSCHRIFT

(11) **EP 4 614 019 B1**
(45) Veröffentlichungstag und Bekanntmachung des Hinweises auf die Patenterteilung: **25.02.2026**
(21) Anmeldenummer: 25158303.5
(22) Anmeldetag: 17.02.2025
(51) Int. Cl.: F16C 32/04

(54) **MAGNETLAGERVORRICHTUNG UND ELEKTROMAGNETISCHER DREHANTRIEB**
MAGNETIC BEARING DEVICE AND ELECTROMAGNETIC ROTARY DRIVE
DISPOSITIF DE PALIER MAGNÉTIQUE ET ACTIONNEUR ROTATIF ÉLECTROMAGNÉTIQUE

(30) Priorität: 05.03.2024 EP 24161380
(43) Veröffentlichungstag der Anmeldung: 10.09.2025
(73) Patentinhaber: Levitronix GmbH, 8048 Zürich (CH)
(72) Erfinder: Steinert, Daniel, 8180 Bülach (CH)
(74) Vertreter: IPS Irsch AG

(56) Entgegenhaltungen:
- EP-A1- 3 232 549
- EP-A1- 4 084 304
- EP-A1- 4 124 845

## Beschreibung

Die Erfindung betrifft eine Magnetlagervorrichtung gemäss dem Oberbegriff des unabhängigen Patentanspruchs und einen elektromagnetischen Drehantrieb mit einer solchen Magnetlagervorrichtung.

Magnetlagervorrichtungen zur berührungslos magnetischen Lagerung eines Rotors haben den Vorteil, dass sie ohne mechanische Lager für den Rotor auskommen. Der Rotor wird mittels magnetischer Kräfte gelagert bzw. stabilisiert, die von einem Stator der Magnetlagervorrichtung generiert werden. Aufgrund der Abwesenheit von mechanischen Lagern eignen sich solche Magnetlagervorrichtungen insbesondere für Pump-, Misch-, Zentrifugier- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive oder aggressive Substanzen gefördert werden, welche mechanische Lager sehr schnell zerstören würden, beispielsweise Pumpen oder Mischer für Slurry, Schwefel-, Phosphorsäure oder andere Chemikalien in der Halbleiterindustrie.

In der biotechnologischen Industrie werden solche Magnetlagervorrichtungen beispielsweise im Zusammenhang mit Bioreaktoren eingesetzt, z. B. bei Zentrifugalpumpen zum Fördern der Fluide in den oder aus dem Bioreaktor, oder bei Mischvorrichtungen, welche die Fluide in dem Bioreaktor durchmischen. In der Halbleiterindustrie werden solche Magnetlagervorrichtung nicht nur für das Fördern aggressiver oder abrasiver Substanzen verwendet, sondern beispielsweise auch für Rotationsvorrichtungen, mit denen Wafer rotiert werden.

Auch ist es bekannt, Magnetlagervorrichtungen für Viskosimeter zu verwenden.

Eine vorteilhafte und an sich bekannte Ausführungsform einer Magnetlagervorrichtung ist die Ausführung in Tempelbauweise, auf die sich auch die vorliegende Erfindung bezieht. Eine gattungsgemäße Magnetlagervorrichtung ist aus EP 4 124 845 A1 bekannt.

Das Charakteristische der Tempelbauweise ist es, dass der Stator der Magnetlagervorrichtung eine Mehrzahl von Spulenkernen aufweist, von denen jeder einen Längsschenkel umfasst, der sich von einem ersten Ende in einer axialen Richtung bis zu einem zweiten Ende erstreckt. Mit der axialen Richtung ist dabei diejenige Richtung gemeint, welche durch die Solldrehachse des Rotors definiert ist, welcher mit der Magnetlagervorrichtung gelagert wird. Die Solldrehachse ist diejenige Drehachse, um welche der Rotor im Betriebszustand rotiert, wenn er bezüglich des Stators in einer zentrierten und unverkippten Position ist. Jeder Spulenkern umfasst zusätzlich zu dem Längsschenkel einen Querschenkel, auch Polstück genannt, welcher jeweils an dem zweiten Ende des Längsschenkels angeordnet ist, und welcher sich in radialer Richtung - üblicherweise nach innen - erstreckt, wobei die radiale Richtung senkrecht zur axialen Richtung ist. Der Querschenkel erstreckt sich also im Wesentlichen rechtwinklig zum Längsschenkel. Die Spulenkerne haben jeweils die Form eines L, wobei die Querschenkel die kurzen Schenkel des L bilden. Der zu lagernde Rotor ist dann zwischen den Querschenkeln angeordnet.

Die Mehrzahl der Längsschenkel, die sich in axialer Richtung erstrecken und an die Säulen eines Tempels erinnern, hat dieser Bauweise ihren Namen gegeben.

In einer Ausführungsform hat der Stator der Magnetlagervorrichtung beispielsweise sechs Spulenkerne, die kreisförmig und äquidistant um eine becherförmige Ausnehmung herum angeordnet sind, in welche der Rotor eingesetzt werden kann. Die ersten Enden der Längsschenkel sind üblicherweise in Umfangsichtung durch einen Rückschluss verbunden, welcher der magnetischen Flussführung dient. Der zu lagernde Rotor umfasst einen magnetisch wirksamen Kern, beispielsweise eine permanentmagnetische Scheibe oder einen permanentmagnetischen Ring, der zwischen den radial innenliegenden Enden der Querschenkel angeordnet ist und im Betriebszustand um die axiale Richtung rotiert, wobei der Rotor berührungslos magnetisch bezüglich des Stators gelagert ist.

Für solche Magnetlagervorrichtungen ist es nicht notwendiger Weise so, dass der magnetisch wirksame Kern des Rotors permanentmagnetisch ausgestaltet sein muss. Es sind auch solche Ausgestaltungen bekannt, bei denen der magnetisch wirksame Kern des Rotors permanentmagnetfrei, also ohne Permanentmagnete ausgestaltet ist. Der magnetisch wirksame Kern des Rotors ist dann beispielsweise ferromagnetisch ausgestaltet und besteht beispielsweise aus Eisen, Nickel-Eisen, Kobalt-Eisen, Silizium-Eisen, Mu-Metall oder einem anderen ferromagnetischen Werkstoff.

Ferner sind Ausgestaltungen möglich, bei denen der magnetisch wirksame Kern des Rotors sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Um die für die berührungslos magnetische Lagerung des Rotors notwendigen elektromagnetischen Felder zu erzeugen, tragen die Längsschenkel Wicklungen. Die Wicklungen sind beispielsweise so ausgestaltet, dass um jeden Längsschenkel herum eine konzentrierte Wicklung gewickelt ist, das heisst, die Spulenachse jeder konzentrierten Wicklung erstreckt sich jeweils in axialer Richtung. Dabei ist es typisch für die Tempelbauweise, dass die Spulenachsen der konzentrierten Wicklungen in der axialen Richtung verlaufen und, dass die konzentrierten Wicklungen nicht in der radialen Ebene angeordnet sind, in welcher der Rotor bzw. der magnetisch wirksame Kern des Rotors im Betriebszustand gelagert wird.

Es sind Ausgestaltungen möglich, bei denen auf jedem Längsschenkel genau eine konzentrierte Wicklung angeordnet ist. In anderen Ausgestaltungen sind auf jedem Längsschenkel mehrere, beispielsweise genau zwei konzentrierte Wicklungen vorgesehen. Auch sind Ausgestaltungen möglich, bei denen Wicklungen vorgesehen sind, die um zwei in Umfangsrichtung benachbarte Längsschenkel herumgewickelt sind, sodass sich diese beiden benachbarten Längsschenkel beide im Innenraum der konzentrierten Wicklung befinden.

Die Spulenkerne, der aus dem Stand der Technik bekannten Magnetlagervorrichtungen sind meist geblecht ausgestaltet. Das bedeutet, dass mehrere Bleche in Form der Spulenkerne gegeneinander isoliert in Umfangsrichtung gestapelt sind. Die geblechte Ausgestaltung der Spulenkerne verhindert Wirbelströme für Magnetfelder, welche in Blechrichtung verlaufen also Felder, welche in axialer Richtung dem Längsschenkel folgen und in radialer Richtung dem Querschenkel folgen.

Für Magnetfelder, welche seitlich, also in Umfangsrichtung aus den Blechen des Längsschenkels und des Querschenkels austreten, ist die Isolation der Bleche unwirksam und es treten somit trotzdem Wirbelströme auf, da diese Magnetfelder orthogonal durch die Bleche hindurchgehen.

Gerade bei Magnetlagervorrichtungen, welche einen grossen magnetischen Spalt, wobei der magnetische Spalt als Abstand zwischen der Stirnfläche des Querschenkels und des magnetisch wirksamen Kerns des Rotors in radialer Richtung definiert ist, aufweisen, werden orthogonale Feldanteile nicht vernachlässigbar und erzeugen merkliche Wirbelstromverluste.

Im Kontext dieser Anmeldung ist mit einem grossen magnetischen Spalt ein magnetischer Spalt gemeint, welcher grösser als 1% des Durchmessers des magnetisch wirksamen Kerns in radialer Richtung ist. In manchen Fällen kann der magnetische Spalt grösser gleich 5% des Durchmessers des magnetisch wirksamen Kerns in radialer Richtung sein.

Ausgehend von diesem Stand der Technik ist es daher eine Aufgabe der Erfindung, eine Magnetlagervorrichtung zur berührungslos magnetischen Lagerung eines Rotors mit einem scheibenförmigen oder ringförmigen magnetisch wirksamen Kern vorzuschlagen, welche geringere Wirbelstromverluste als der bisherige Stand der Technik aufweist.

Ferner ist es eine Aufgabe der Erfindung, einen elektromagnetischen Drehantrieb mit einer solchen Magnetlagervorrichtung vorzuschlagen.

Der diese Aufgabe lösende Gegenstand der Erfindung ist durch die Merkmale des unabhängigen Patentanspruchs gekennzeichnet.

Erfindungsgemäss wird also eine Magnetlagervorrichtung vorgeschlagen, zur berührungslos magnetischen Lagerung eines Rotors, der einen scheibenförmigen oder ringförmigen magnetisch wirksamen Kern umfasst, wobei die Magnetlagervorrichtung einen Stator aufweist, welcher eine Mehrzahl von Spulenkernen umfasst, wobei jeder Spulenkern geblecht aus Elementen hergestellt ist, wobei die Elemente in Umfangsrichtung des Stators gestapelt sind, wobei jeder Spulenkern eine erste laterale Begrenzungsfläche und eine zweite laterale Begrenzungsfläche aufweist, wobei jeder Spulenkern einen Längsschenkel umfasst, welcher sich von einem ersten Ende in einer axialen Richtung bis zu einem zweiten Ende erstreckt, sowie einen Querschenkel, welcher an dem zweiten Ende des Längsschenkels angeordnet ist, und sich in einer radialen Richtung erstreckt, die senkrecht zur axialen Richtung ist, wobei an jedem Längsschenkel mindestens eine konzentrierte Wicklung vorgesehen ist, welche den jeweiligen Längsschenkel umgibt, wobei der Stator ferner eine becherförmige Ausnehmung aufweist, in welche der Rotor einsetzbar ist, wobei die becherförmige Ausnehmung an einem axialen Ende des Stators angeordnet ist, wobei die Querschenkel um die becherförmige Ausnehmung herum angeordnet sind und wobei mindestens eine der ersten oder der zweiten lateralen Begrenzungsflächen mindestens einen Schlitz aufweist.

Das Einbringen von Schlitzen in mindestens eine der beiden lateralen Begrenzungsflächen des Spulenkerns stellt eine elektrische Isolation dar. Das heisst, der mindestens eine Schlitz sorgt dafür, dass der Pfad der Wirbelströme im Spulenkern unterbrochen und somit blockiert wird. Dies hat zur Folge, dass lediglich kleine Wirbelströme im Spulenkern verbleiben und die Wirbelstromverluste im Spulenkern insgesamt drastisch reduziert werden. Es ist dabei vorteilhaft, dass der mindestens eine Schlitz parallel oder zumindest näherungsweise parallel zum Verlauf des magnetischen Felds im Spulenkern verlaufen sollte, um diesen nicht zu blockieren.

Die Fertigung der Schlitze, kann über diverse Methoden erfolgen. Zu diesen zählen unter anderem mechanische Verfahren, wie z.B. Fräsen, Stanzen oder Schneiden, wobei zu letzterem auch der Einsatz von Lasern oder von Wasserstrahlschneidern zählt.

Gemäss einer bevorzugten Ausgestaltung sind die Elemente aus Elektroblech hergestellt.

Unter einem Elektroblech wird nach der allgemeinen Definition ein weichmagnetischer Werkstoff für Magnetkerne verstanden. Ebenso besteht die Möglichkeit Mu-Metall zu verwenden.

Gemäss einer bevorzugten Ausgestaltung erstreckt sich der mindestens eine Schlitz nur im Querschenkel. Für Anwendungen, in denen der Spulenkern eine hohe Stabilität aufweisen muss, kann es vorteilhaft sein, dass sich der mindestens eine Schlitz nur im Querschenkel erstreckt. Auch durch diese Ausgestaltung werden einige Wirbelstromverluste verringert da die meisten Effekte, die zu den Wirbelstromverlusten führen im Bereich des zweiten Endes auftreten.

Gemäss einer bevorzugten Ausgestaltung erstreckt sich der mindestens eine Schlitz im Querschenkel und im Längsschenkel. Dies ist vorteilhaft, um die Wirbelstromverluste im Spulenkern weiter reduzieren zu können. Wie weit sich der mindestens eine Schlitz im Längsschenkel in axialer Richtung in Richtung des ersten Endes des Längsschenkels erstreckt ist variabel. Von einer geringen Erstreckung von 5% der Gesamtlänge des Längsschenkels in axialer Richtung bis zu einer Erstreckung hin zum ersten Ende des Längsschenkels sind alle Länge des mindestens einen Schlitzes möglich.

In einer bevorzugten Ausgestaltung weist der mindestens eine Schlitz eine Rundung auf, welche den Schlitz aus der radialen Richtung in die axiale Richtung umlenkt.

Gemäss einer bevorzugten Ausgestaltung erstreckt sich der mindestens eine Schlitz von der ersten lateralen Begrenzungsfläche bis zu der zweiten lateralen Begrenzungsfläche.

In einer bevorzugten Ausgestaltung ist die Erstreckung des mindestens einen Schlitzes in Umfangsrichtung des Stators gesehen kürzer als der Abstand der ersten lateralen Begrenzungsfläche von der zweiten lateralen Begrenzungsfläche.

In anderen Worten, der mindestens eine Schlitz erstreckt sich nicht durch den gesamten Spulenkern in Umfangsrichtung des Stators. Das heisst der mindestens eine Schlitz ist nicht in allen Elementen der geblechten Ausgestaltung des Spulenkerns vorgesehen. Dies ist vorteilhaft, da gerade in den Elementen, welche direkt bzw. nahe an den beiden lateralen Begrenzungsflächen angeordnet sind, der Grossteil der Wirbelströme entsteht. Somit werden durch den mindestens einen Schlitz die Pfade der Wirbelströme im Spulenkern dort unterbrochen, wo sie am häufigsten auftreten. Dies sorgt für eine deutliche Reduktion der Wirbelstromverluste. Des Weiteren ist diese Ausgestaltung von Vorteil für die Stabilität des Spulenkerns.

Gemäss einer bevorzugten Ausgestaltung sind mehrere Schlitze vorgesehen, welche parallel oder zumindest näherungsweise parallel zueinander angeordnet sind.

Die Anordnung der mehreren Schlitze parallel oder zumindest näherungsweise parallel zueinander ist vorteilhaft, da sie dadurch auch parallel oder zumindest näherungsweise parallel zum Verlauf des magnetischen Felds im Spulenkern verlaufen, sodass sie diesen nicht blockieren.

Ferner ist es bevorzugt, dass der Spulenkern an einem axial oberen Ende eine Abrundung aufweist, welche den Spulenkern aus der axialen Richtung in die radiale Richtung umlenkt.

Als Beispiel, im Fall eines L-Förmigen Spulenkerns, wobei das lange Stück des "L" durch den Längsschenkel und das kurze Stück des "L" durch den Querschenkel gebildet wird, ist die von der becherförmigen Ausnehmung betrachtet radial aussen liegenden Kante des Querschenkels, die sich in der radialen Ebene in Umfangsrichtung erstreckt, abgerundet ausgestaltet. Diese Ausgestaltung hat den Vorteil, dass sie geringere Wirbelstromverluste aufweist und auch im Hinblick auf die Konstruktion einfacher realisierbar ist.

In einer bevorzugten Ausgestaltung ist am ersten Ende der Längsschenkel ein Rückschluss angeordnet, der die ersten Enden aller Längsschenkel verbindet, wobei der Rückschluss ringförmig mit einem metallischen Band ausgestaltet ist, welches sich von einem radial inneren Anfang zu einem radial äusseren Ende erstreckt, wobei das Band mehrere Bandwicklungen formt, die bezüglich der radialen Richtung flächig aneinander anliegen.

Gemäss einer bevorzugten Ausgestaltung sind an jedem Längsschenkel zwei konzentrierte Wicklungen vorgesehen, von denen jede den jeweiligen Längsschenkel umgibt, und die bezüglich der axialen Richtung benachbart zueinander angeordnet sind.

Ferner ist es bevorzugt, dass sowohl in der ersten lateralen Begrenzungsfläche als auch in der zweiten lateralen Begrenzungsfläche jeweils mindestens ein Schlitz vorgesehen ist.

Dies ist vorteilhaft, da gerade in den Elementen, welche direkt an den beiden lateralen Begrenzungsflächen angeordnet sind der Grossteil der Wirbelströme entsteht. Somit werden durch den jeweils mindestens einen Schlitz die Pfade der Wirbelströme im Spulenkern dort unterbrochen, wo sie am häufigsten auftreten. Dies sorgt für eine deutliche Reduktion der Wirbelstromverluste.

Gemäss einer besonders bevorzugten Ausführungsform ist der Stator der Magnetlagervorrichtung zur Erzeugung eines Drehmoments ausgestaltet, mit welchem der Rotor berührungslos magnetisch zur Rotation um die axiale Richtung antreibbar ist.

Hierbei ist der Stator als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine Solldrehachse bewirkt, und welches zum anderen eine beliebig einstellbare Querkraft auf den Rotor ausübt, sodass dessen radiale Position aktiv steuerbar bzw. regelbar ist.

Durch die Erfindung wird ferner ein elektromagnetischer Drehantrieb vorgeschlagen, der als Tempelmotor ausgestaltet ist, wobei der elektromagnetische Drehantrieb eine erfindungsgemässe Magnetlagervorrichtung umfasst, sowie einen Rotor mit einem scheibenförmigen oder ringförmigen magnetisch wirksamen Kern, wobei der Rotor in die becherförmige Ausnehmung einsetzbar ist, und wobei der Rotor als Rotor des elektromagnetischen Drehantriebs ausgestaltet ist.

Solche elektromagnetischen Drehantriebe sind auch unter dem Begriff des lagerlosen Motors bekannt. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen:
- Fig. 1:: eine perspektivische Darstellung eines ersten Ausführungsbeispiels einer erfindungsgemässen Magnetlagervorrichtung,
- Fig. 2:: eine perspektivische Darstellung eines einzelnen Spulenkerns der Magnetlagerlagervorrichtung aus Fig. 1,
- Fig. 3 - Fig. 6: verschiedene Varianten für die Ausgestaltung eines Spulenkerns, jeweils in einer perspektivischen Darstellung,
- Fig. 7:: eine perspektivische Darstellung eines zweiten Ausführungsbeispiels einer erfindungsgemässen Magnetlagervorrichtung, und
- Fig. 8:: eine schematische Schnittdarstellung für eine Ausgestaltung eines Statorgehäuses.

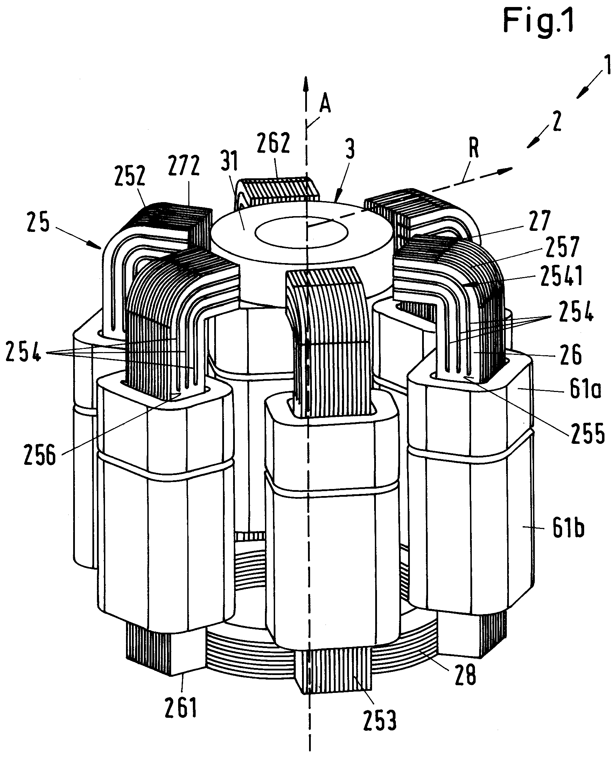

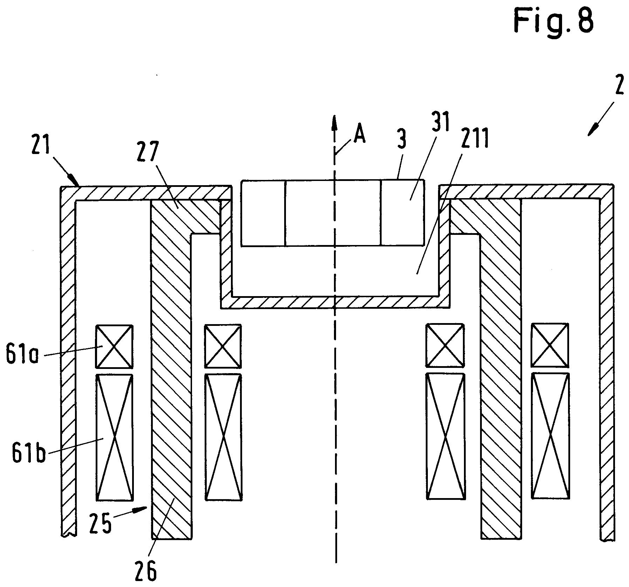

Fig. 1 zeigt eine perspektivische Darstellung eines Ausführungsbeispiels einer erfindungsgemässen Magnetlagervorrichtung 1, die gesamthaft mit dem Bezugszeichen 1 bezeichnet ist. Die Magnetlagervorrichtung 1 ist für die berührungslos magnetische Lagerung eines Rotors 3 ausgestaltet, der einen scheibenförmigen oder ringförmigen magnetisch wirksamen Kern 31 umfasst. Die Magnetlagervorrichtung 1 ist gemäss der Tempelbauweise ausgestaltet und umfasst einen Stator 2. Üblicherweise umfasst der Stator 2 ein Statorgehäuse 21 (Fig. 8), welches in Fig. 1 aus Gründen der besseren Übersicht allerdings nicht dargestellt ist. Daher veranschaulicht Fig. 8 in einer schematischen Schnittdarstellung eine Ausgestaltung eines Statorgehäuses 21.

An einem axialen Ende des Statorgehäuses 21 ist eine becherförmige Ausnehmung 211 vorgesehen in die der Rotor 3 einsetzbar ist. Der Rotor 3 ist zur Rotation um eine Solldrehachse ausgestaltet. Diese Solldrehachse definiert eine axiale Richtung A. Üblicherweise stimmt die Mittelachse des Stators 2, welche sich in der axialen Richtung A erstreckt, mit der Solldrehachse überein. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet, so wie dies in Fig. 1 dargestellt ist.

Der Stator 2 weist eine Mehrzahl von Spulenkernen 25 - hier sechs Spulenkerne 25 - auf wobei jeder Spulenkern 25 geblecht aus Elementen 253 hergestellt ist. Die Elemente 253 sind in Umfangsrichtung des Stators 2 gestapelt, und jeder Spulenkern 25 weist eine erste laterale Begrenzungsfläche 255 und eine zweite laterale Begrenzungsfläche 256 auf. Mit Umfangsrichtung ist die Richtung gemeint, welche senkrecht auf der radialen Richtung R und senkrecht auf der axialen Richtung A steht. Des Weiteren umfasst jeder Spulenkern 25 einen Längsschenkel 26, welcher sich von einem ersten Ende 261 in einer axialen Richtung A bis zu einem zweiten Ende 262 erstreckt, sowie einen Querschenkel 27, welcher an dem zweiten Ende 262 des Längsschenkels angeordnet ist, und sich in einer radialen Richtung erstreckt, die senkrecht zur axialen Richtung A ist.

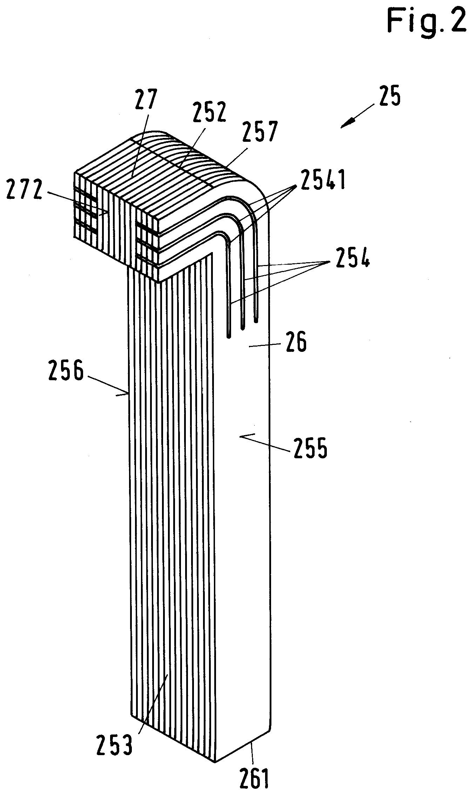

Zum besseren Verständnis ist in Fig. 2 eine perspektivische Darstellung eines einzelnen Spulenkerns 25 der Magnetlagerlagervorrichtung 1 aus Fig. 1 dargestellt.

Die Spulenkerne 25 des Stators 2 sind äquidistant auf einer Kreislinie angeordnet, sodass die Querschenkel 27 den magnetisch wirksamen Kern 31 des Rotors 3 umgeben, wenn der Rotor 3 in die becherförmige Ausnehmung 211 eingesetzt ist.

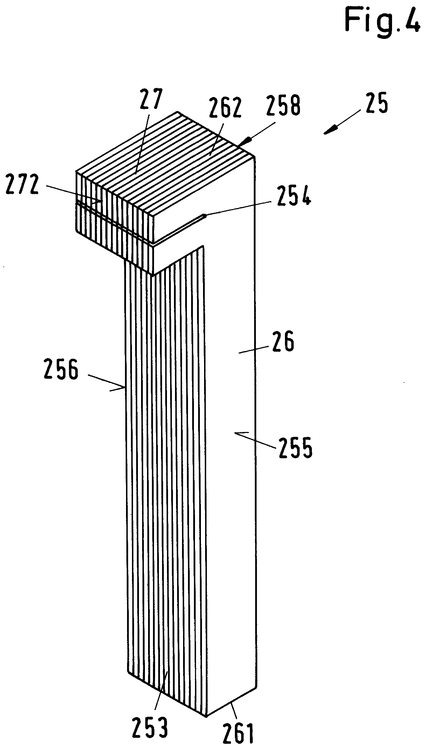

In diesem Ausführungsbeispiel weisen die Spulenkerne 25 an einem axial oberen Ende 252 eine Abrundung 257 auf, welche den Spulenkern 25 aus der axialen Richtung A in die radiale Richtung R umlenkt. Die Spulenkerne 25 weisen eine Abrundung 257 der äusseren Kante 258 (Fig. 4) des Spulenkerns 25 am axial oberen Ende des Spulenkerns 252 auf. Das heisst in diesem Ausführungsbeispiel ist die radial aussen liegende Kante 258 am axial oberen Ende 252 des Spulenkerns 25 eine gebrochene Kante bzw. eine abgerundete Kante, wie durch die Abrundung 257 dargestellt. Je nach Ausführungsbeispiel des Spulenkerns 25 kann sich die Abrundung 257 nur in Bereichen des Längsschenkels 26 oder nur in Bereichen des Querschenkels 27 oder aber sowohl in Bereichen des Längsschenkels 26 wie auch des Querschenkels 27 erstrecken. Dies hat den Vorteil, dass diese Ausgestaltung des Spulenkerns 25 geringere Wirbelstromverluste aufweist und auch im Hinblick auf die Konstruktion einfacher umzusetzen ist.

An jedem Längsschenkel 26 ist mindestens eine konzentrierte Wicklung 61 angeordnet, welche den jeweiligen Längsschenkel 26 umgibt. In anderen Ausführungsformen können an den Längsschenkeln 26 auch mehr als eine konzentrierte Wicklung 61 angeordnet sein. Beispielsweise gibt es Ausführungsbeispiele, wie hier in Fig. 1 dargestellt, bei denen an jedem der Längsschenkel 26 jeweils genau zwei konzentrierte Wicklungen 61a, 61b vorgesehen sind, von denen jede den jeweiligen Längsschenkel 26 umgibt, wobei die beiden auf dem gleichen Längsschenkel 26 angeordneten Wicklungen 61a, 61b bezüglich der axialen Richtung A benachbart zueinander angeordnet sind.

Die konzentrierten Wicklungen 61a, 61b dienen dazu, elektromagnetische Felder zu erzeugen, mit welchen der Rotor 3 berührungslos magnetisch in der becherförmigen Ausnehmung 211 (Fig. 8) lagerbar ist.

Die Elemente 253 können aus Elektroblech hergestellt sein. Unter einem Elektroblech wird nach der allgemeinen Definition ein weichmagnetischer Werkstoff für Magnetkerne verstanden. Ebenso besteht die Möglichkeit Mu-Metall für das Band zu verwenden.

Die Anzahl der Elemente 253 in allen Ausführungsbeispielen und Figuren ist rein exemplarisch zu verstehen. Die Anzahl kann grösser oder aber auch kleiner sein als dargestellt.

In dem in Fig. 1 dargestellten ersten Ausführungsbeispiel der erfindungsgemässen Magnetlagervorrichtung 1 sind sowohl in der ersten wie auch in der zweiten lateralen Begrenzungsflächen 255, 256 jeweils drei Schlitze 254 angeordnet. Die Schlitze 254 erstrecken sich sowohl im Querschenkel 27 wie auch im Längsschenkel 26. Das Einbringen der Schlitze 254 in die beiden lateralen Begrenzungsflächen 255, 256 des Spulenkerns 25 stellt eine elektrische Isolation dar. Das heisst, die Schlitze 254 sorgen dafür, dass der Pfad der Wirbelströme im Spulenkern 25 unterbrochen und somit blockiert werden. Dies hat zur Folge, dass lediglich kleine Wirbelströme im Spulenkern 25 verbleiben und die Wirbelstromverluste im Spulenkern 25 insgesamt drastisch reduziert werden.

Jeder der Schlitze 254 weist in diesem Ausführungsbeispiel eine Rundung 2541 auf, welche den jeweiligen Schlitz 254 aus der radialen Richtung R in die axiale Richtung A umlenkt. Die Schlitze 254 sind parallel oder zumindest näherungsweise parallel zueinander und parallel oder zumindest näherungsweise parallel zum Verlauf des magnetischen Felds im Spulenkern 25 angeordnet. Dies hat den Vorteil, dass die Schlitze 254 dadurch den Verlauf des magnetischen Felds im Spulenkern 25 nicht behindern und/oder blockieren.

Die Schlitze 254 erstrecken sich hierbei nicht über die gesamte Erstreckung des Längsschenkels 26 in axialer Richtung A, sondern nur zu einem Teil und enden vor einem axial oberen Ende der konzentrierten Wicklung 61a.

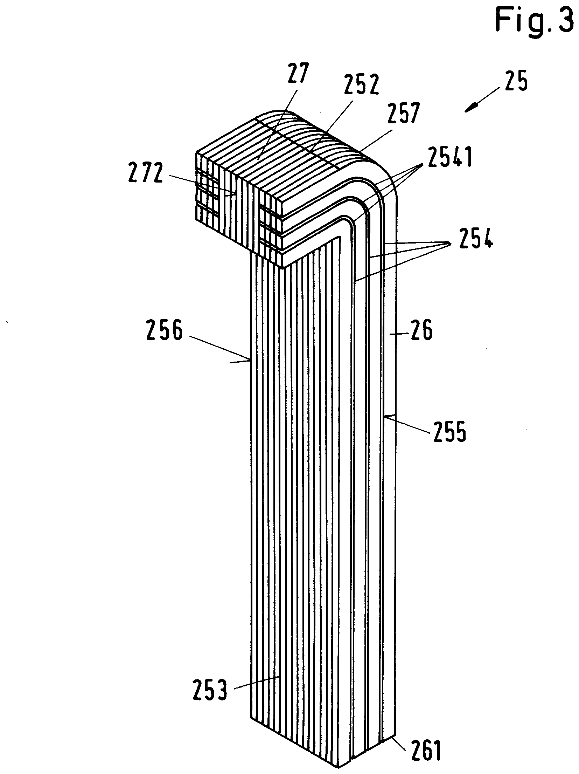

Des Weiteren sind aber auch Ausgestaltungen möglich, in denen der mindestens eine Schlitz 254 eine längere Erstreckung im Längsschenkel 26 aufweist als in der in Fig. 2 dargestellten Ausgestaltung. Solch eine mögliche Ausgestaltung ist in Fig. 3 dargestellt. Die dortige perspektivische Darstellung einer Variante für die Ausgestaltung eines Spulenkerns 25, zeigt die maximal mögliche Erstreckung eines Schlitzes 254 in einem Spulenkern 25.

Es ist natürlich möglich, dass die Schlitze 254 jegliche Erstreckungslängen im Längsschenkel 26 aufweisen können. Es ist ebenfalls möglich, dass sich die Schlitze 254 auch nur im Querschenkel 27 erstrecken.

In diesem Ausführungsbeispiel ist die Erstreckung der Schlitze 254 in Umfangsrichtung des Stators 2 gesehen kürzer als der Abstand der ersten lateralen Begrenzungsfläche 255 von der zweiten lateralen Begrenzungsfläche 256. In anderen Worten, die Schlitze 254 durchdringen nicht alle Elemente 253 der Spulenkerne 25, sondern nur eine bestimmte Anzahl. Im hier vorliegenden Ausführungsbeispiel sind es pro Spulenkern 25 acht Elemente 253, also von jeder lateralen Begrenzungsfläche 255, 256 aus gesehen jeweils vier Elemente 253.

Dies ist vorteilhaft, da gerade in den Elementen 253, welche direkt bzw. nahe an den beiden lateralen Begrenzungsflächen 255, 256 angeordnet sind, der Grossteil der Wirbelströme entsteht. Somit werden durch die Schlitz 254 die Pfade der Wirbelströme im Spulenkern 25 dort unterbrochen, wo sie am häufigsten auftreten. Dies sorgt für eine deutliche Reduktion der Wirbelstromverluste. Des Weiteren ist eine nicht komplette Durchdringung der Schlitze 254 durch alle Elemente 253 des Spulenkerns 25 für die Stabilität des Spulenkerns 25 von Vorteil.

Es sind aber auch Ausgestaltung möglich, bei denen sich die Schlitze 254 von der ersten lateralen Begrenzungsfläche 255 bis zu der zweiten lateralen Begrenzungsfläche 256 erstrecken.

Gemäss einer speziell bevorzugten Ausgestaltung ist der Stator 2 so ausgestaltet, dass er zusätzlich zur berührungslos magnetischen Lagerung des Rotors 3 auch ein Drehmoment auf den Rotor 3 bzw. den magnetisch wirksamen Kern 31 des Rotors 3 ausüben kann, welches den Rotor 3 zu einer Rotation um die Solldrehachse antreibt. Das heisst, bei dieser bevorzugten Ausgestaltung ist der Rotor 3 zur Rotation um die axiale Richtung A antreibbar.

Bei dieser Ausgestaltung erzeugen die konzentrierten Wicklungen 61 also elektromagnetische Drehfelder, mit denen der Rotor 3 sowohl berührungslos magnetisch bezüglich des Stators 2 lagerbar ist als auch berührungslos zur Rotation um die axiale Richtung A antreibbar ist.

Es versteht sich, dass die Anzahl von sechs Spulenkernen 25 zwar bevorzugt, aber nur beispielhaft zu verstehen ist. Es sind natürlich auch solche Ausgestaltungen möglich, bei denen der Stator 2 weniger als sechs, z. B. fünf oder vier oder drei Spulenkerne 25 aufweist, oder solche Ausgestaltungen, bei denen der Stator 2 mehr als sechs, z. B. sieben oder acht oder neun oder zwölf Spulenkerne 25 aufweist oder eine beliebige grössere Anzahl von Spulenkernen 25.

Der Rotor 3 umfasst den magnetisch wirksamen Kern 31, welcher ring- oder scheibenförmig ausgestaltet ist. Der magnetisch wirksame Kern 31 ist gemäss der Darstellung in Fig. 1 als Ring ausgestaltet und definiert eine magnetische Mittelebene. Alternativ kann der magnetisch wirksame Kern 31 auch als Scheibe ausgestaltet sein. In der Regel ist bei einem scheibenförmigen oder ringförmigen magnetisch wirksamen Kern 31 die magnetische Mittelebene die geometrische Mittelebene des magnetisch wirksamen Kerns 31 des Rotors 3, die senkrecht zur axialen Richtung A liegt. Im Betriebszustand ist der magnetisch wirksame Kern 31 in einer radialen Ebene gelagert, welche senkrecht auf der axialen Richtung A steht.

Die radiale Ebene ist in Fig. 1 durch die Linie einer radialen Richtung R angedeutet, die senkrecht auf der axialen Richtung A steht. Die radiale Ebene ist diejenige Ebene, welche senkrecht auf der axialen Richtung A steht und eine radiale Richtung R enthält. Die radiale Ebene ist diejenige Ebene, in welcher der magnetisch wirksame Kern 31 des Rotors 3 im Betriebszustand zwischen den Stirnflächen 272 im Stator 2 aktiv magnetisch gelagert ist. Wenn der Rotor 3 nicht verkippt und in axialer Richtung A nicht ausgelenkt ist, liegt die magnetische Mittelebene in der radialen Ebene. Die radiale Ebene definiert die x-y-Ebene eines kartesischen Koordinatensystems, dessen z-Achse in axialer Richtung A verläuft.

Mit der radialen Position des magnetisch wirksamen Kerns 31 bzw. des Rotors 3 wird die Lage des Rotors 3 in der radialen Ebene bezeichnet.

Da es für das Verständnis der Erfindung ausreichend ist, ist in Fig. 1 von dem Rotor 3 nur der magnetisch wirksame Kern 31 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder aus einem Metall oder aus einer Metalllegierung oder aus einer Keramik bzw. einem keramischen Werkstoff. Ferner kann der Rotor 3 auch Flügel zum Mischen, Rühren oder Pumpen von Fluiden oder sonstige Komponenten umfassen.

Wenn der Rotor 3 in die becherförmige Ausnehmung 211 (Fig. 8) eingesetzt ist, wird der Rotor 3 und insbesondere der magnetisch wirksame Kern 31 des Rotors 3 von den radial aussenliegend angeordneten Stirnflächen 272 der Querschenkel 27 der Spulenkerne 25 des Stators 2 umgeben. Die Querschenkel 27 bilden somit eine Mehrzahl von ausgeprägten Statorpolen- hier sechs Statorpole.

Wenn sich der magnetisch wirksame Kern 31 des Rotors 3 während des Betriebs in seiner Soll-Position befindet, so ist der magnetisch wirksame Kern 31 zwischen den Stirnflächen 272 der Querschenkel 27 zentriert. Die konzentrierten Wicklungen 61 sind darstellungsgemäss unterhalb der radialen Ebene angeordnet und so ausgerichtet, dass ihre Spulenachsen in axialer Richtung A verlaufen.

Alle ersten Enden 261 der Längsschenkel 26 - also die darstellungsgemäss (Fig. 1) unteren Enden 261 - sind durch einen Rückschluss 28 miteinander verbunden. Der Rückschluss 28 ist vorzugsweise ringförmig ausgestaltet. Dabei sind solche Ausgestaltungen möglich (siehe z. B. Fig. 1), bei welchen sich der Rückschluss 28 radial innenliegend entlang aller ersten Enden 261 der Längsschenkel 26 erstreckt.

Um die für die magnetische Lagerung des Rotors 3 und optional zur Erzeugung eines Drehmoments auf den Rotor 3 notwendigen elektromagnetischen Felder zu erzeugen, tragen die Längsschenkel 26 der Spulenkerne 25 die als konzentrierte Wicklungen 61 ausgestalteten Wicklungen.

Mit diesen konzentrierten Wicklungen 61 werden im Betriebszustand diejenigen elektromagnetischen Drehfelder erzeugt, mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene, aktiv steuerbar bzw. regelbar ist. Optional wird mit diesen elektromagnetischen Drehfeldern zusätzlich ein Drehmoment auf den Rotor 3 bewirkt.

Mit dem "magnetisch wirksamen Kern 31" des Rotors 3 ist derjenige Bereich des Rotors 3 gemeint, welcher für die Erzeugung der magnetischen Lagerkräfte und optional für die Drehmomentbildung magnetisch mit dem Stator 2 zusammenwirkt.

Wie bereits erwähnt, ist der magnetisch wirksame Kern 31 bei diesem Ausführungsbeispiel ringförmig ausgestaltet. Ferner ist der magnetisch wirksame Kern 31 permanentmagnetisch ausgestaltet. Dazu kann der magnetisch wirksame Kern 31 mindestens einen Permanentmagneten, aber auch mehrere Permanentmagnete umfassen oder - wie im hier beschriebenen Ausführungsbeispiel - vollständig aus einem permanentmagnetischen Material bestehen, sodass der magnetisch wirksame Kern 31 der Permanentmagnet ist. Der magnetisch wirksame Kern 31 ist beispielsweise in radialer Richtung magnetisiert.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10'000 A/m beträgt.

Es sind auch solche Ausgestaltungen möglich, bei denen der magnetisch wirksame Kern 31 permanentmagnetfrei, also ohne Permanentmagnete ausgestaltet ist. Der Rotor 3 ist dann z.B. als Reluktanzläufer ausgestaltet. Der magnetisch wirksame Kern 31 des Rotors 3 besteht dann beispielsweise aus einem weichmagnetischen Material. Geeignete weichmagnetische Materialien für den magnetisch wirksamen Kern 31 sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 31 des Rotors 3 sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Auch sind Ausgestaltungen möglich, bei denen der Rotor nach dem Prinzip eines Käfigläufers ausgestaltet ist.

Der ringförmige Rückschluss 28 kann aus einem weichmagnetischen Material gefertigt sein, weil es als Flussleitelemente zur Führung des magnetischen Flusses dient. Ebenso ist es möglich, dass auch die Spulenkerne 25 des Stators 2 aus einem weichmagnetischen Material gefertigt sind.

Geeignete weichmagnetische Materialien für die Spulenkerne 25 und den Rückschluss 28 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 2 eine Ausgestaltung als Statorblechpaket bevorzugt, bei welcher der Rückschluss 28, geblecht ausgestaltet ist, das heisst er besteht aus mehreren dünnen Blechelementen, die parallel zueinander in axialer Richtung A aufeinandergestapelt sind. Alle Flussleitelemente sind identisch ausgestaltet, hier also jeweils im Wesentlichen ringförmig und auch mit der gleichen Dicke. Somit ist der Rückschluss 28 selbst im Wesentlichen ringförmig ausgestaltet und erstreckt sich im zusammengesetzten Zustand radial innenliegend entlang der ersten Enden 261 der Längsschenkel 26.

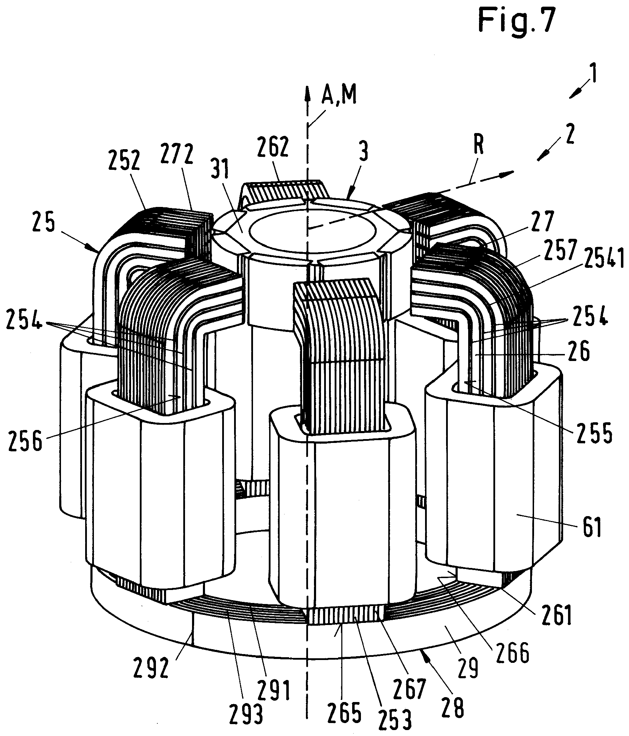

Ebenso sind Ausgestaltungen möglich bei welchen als Rückschluss 28 ein sogenannter Ringbandkern verwendet wird. Dabei handelt es sich um ein aufgewickeltes Band 29. Solch eine Ausgestaltung ist in dem zweiten Ausführungsbeispiel realisiert, das in Fig. 7 dargestellt ist.

Ferner ist es möglich, dass der Rückschluss 28 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator 2 auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

Während des Betriebs der Magnetlagervorrichtung 1 wirkt der magnetisch wirksame Kern 31 des Rotors 3 mit dem Stator 2 zusammen, derart, dass der Rotor 3 berührungslos magnetisch bezüglich des Stators 2 lagerbar ist, und vorzugsweise zusätzlich berührungslos magnetisch in Rotation um die axiale Richtung A versetzt werden kann. Dabei ist es besonders vorteilhaft, dass dieselben Wicklungen 61 mit denen die magnetische Lagerung des Rotors 3 bewirkt wird, auch zur Erzeugung eines Drehmoments auf den Rotor 3 dienen. Vorzugsweise sind dann drei Freiheitsgrade des Rotors 3, nämlich seine Position in der radialen Ebene und seine Rotation, aktiv regelbar. Bezüglich seiner axialen Auslenkung aus der radialen Ebene in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene ist der magnetisch wirksame Kern 31 des Rotors 3 ebenfalls passiv magnetisch stabilisiert. Der Rotor 3 ist also durch das Zusammenwirken des magnetisch wirksamen Kerns 31 mit den Spulenkernen 25 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist, bezeichnet auch im Rahmen dieser Anmeldung eine aktive magnetische Lagerung eine solche, die aktiv steuer- bzw. regelbar ist, beispielsweise über die mit den konzentrierten Wicklungen 61 generierten elektromagnetischen Felder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Bei der Magnetlagervorrichtung 1 wird im Unterschied zu klassischen Magnetlagern die magnetische Lagerung - und optional die Generierung eines auf den Rotor wirkenden Drehmoments - über elektromagnetische Drehfelder realisiert. Zur kombinierten Erzeugung der magnetischen Lagerkräfte und eines Drehmoments zum Rotieren des Rotors 3 um die axiale Richtung A ist es einerseits möglich - wie in Fig 7 gezeigt-, an jedem Längsschenkel 26 genau eine konzentrierte Wicklung 61 anzuordnen.

Andererseits sind auch Ausgestaltungen möglich, bei denen zur kombinierten Erzeugung der magnetischen Lagerkräfte und eines Drehmoments zum Rotieren des Rotors 3 zwei unterschiedliche Wicklungssysteme vorgesehen sind. Dazu sind dann beispielsweise, wie in Fig. 1 dargestellt, an jedem Längsschenkel 26 jeweils genau zwei konzentrierte Wicklungen 61a, 61b angeordnet, die bezüglich der axialen Richtung A benachbart zueinander angeordnet sind. Eine dieser beiden Wicklungen 61a, 61b gehört zu dem ersten der beiden Wicklungssystem und die andere zu dem zweiten der beiden Wicklungssysteme.

Bei der in Fig. 7 dargestellten Ausführungsform mit genau einer konzentrierten Wicklung 61 an jedem Spulenkern 25 werden beispielsweise die in einer Kontrolleinheit jeweils ermittelten Werte für den für die Lagerung benötigten Strom und den für die Erzeugung des Drehmoments benötigten Strom rechnerisch -also z. B. mit Hilfe von Software - addiert bzw. überlagert. Der sich daraus ergebende Gesamtstrom wird dann in die jeweilige konzentrierte Wicklung 61 eingeprägt.

Ist der Stator 2 der erfindungsgemässen Magnetlagervorrichtung 1 zur Erzeugung eines Drehmoments ausgestaltet, so ist die Magnetlagervorrichtung 1 für einen elektromagnetischen Drehantrieb, der als Tempelmotor ausgestaltet ist, geeignet. Ebenso ist es möglich, dass die erfindungsgemässe Magnetlagervorrichtung 1 auch für andere Vorrichtungen wie z.B. Zentrifugalpumpen, Mischvorrichtungen zum Mischen fliessfähiger Substanzen, Rührvorrichtungen, beispielsweise zum Durchmischen eines Fluids in einem Tank, Lüfter oder auch Vorrichtungen zum Tragen und Rotieren von Wafern, beispielsweise in der Halbleiterfertigung, geeignet ist.

Fig. 4 zeigt eine perspektivische Darstellung einer weiteren Variante für die Ausgestaltung eines Spulenkerns 25. In dieser Variante ist der Spulenkern 25 an der Kante 258 nicht abgerundet, sondern rechteckig ausgestaltet. In diesem Ausführungsbeispiel erstreckt sich nur ein Schlitz 254 im Querschenkel 27, im Längsschenkel 26 ist keine Erstreckung des Schlitzes 254. Der Schlitz 254 erstreckt sich von der ersten lateralen Begrenzungsfläche 255 bis zu der zweiten lateralen Begrenzungsfläche 256, d.h. er durchdringt alle Elemente 253 des Spulenkerns 25.

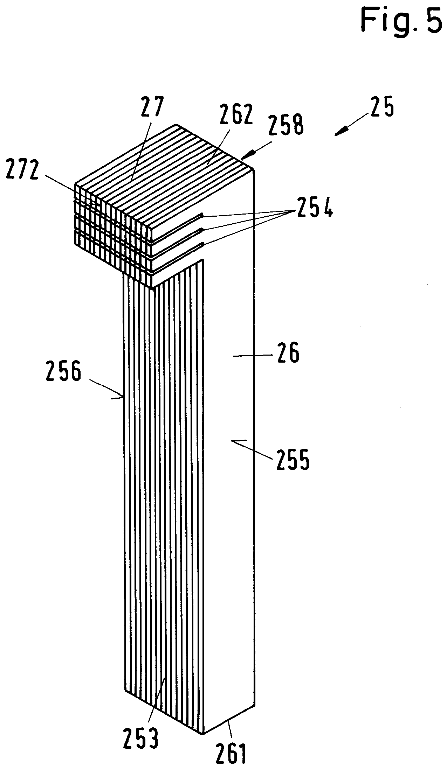

Fig. 5 zeigt eine perspektivische Darstellung einer weiteren Variante für die Ausgestaltung eines Spulenkerns 25. Ein Unterschied zu der Variante in Fig. 4 ist, dass in dieser Variante drei Schlitze 254 vorhanden sind, welche sich alle von der ersten lateralen Begrenzungsfläche 255 bis zu der zweiten lateralen Begrenzungsfläche 256 erstrecken.

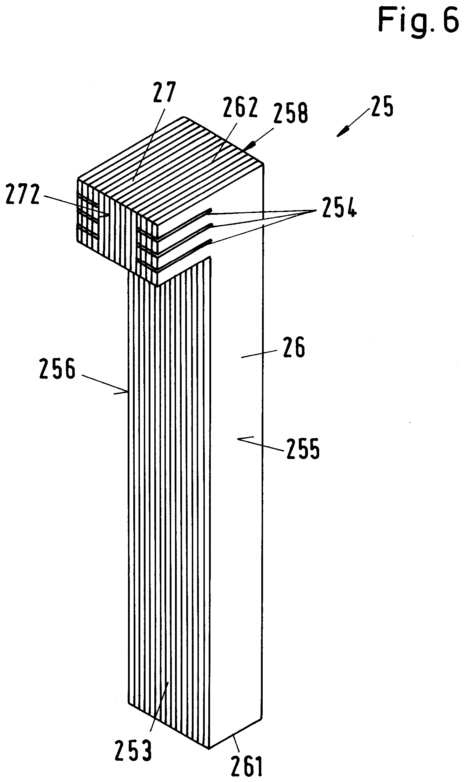

Fig. 6 zeigt eine perspektivische Darstellung einer weiteren Variante für die Ausgestaltung eines Spulenkerns 25. Hierbei ist der Unterschied zu der Variante aus Fig. 4 einerseits, dass drei Schlitze 254 vorhanden sind und andererseits die Erstreckung der drei Schlitze 254 in Umfangsrichtung des Stators 2 gesehen kürzer ist als der Abstand der ersten lateralen Begrenzungsfläche von der zweiten lateralen Begrenzungsfläche. Das heisst, die Schlitze 254 durchdringen nicht alle Elemente 253 der Spulenkerne 25 sondern nur eine bestimmte Anzahl. Im hier vorliegenden Ausführungsbeispiel sind es acht Elemente 253, also von jeder lateralen Begrenzungsfläche 255, 256 aus gesehen jeweils vier Elemente 253.

Selbstverständlich sind auch die beschriebenen Varianten und Ausführungsbeispiele der Spulenkerne 25 aus den Figuren 2-6 untereinander beliebig in jeglicher Form kombinierbar. Ebenso gelten alle Erläuterungen in gleicher Weise oder sinngemäss gleich für alle Varianten.

Fig. 7 zeigt eine perspektivische Darstellung eines zweiten Ausführungsbeispiels einer erfindungsgemässen Magnetlagervorrichtung 1. In der folgenden Beschreibung des zweiten Ausführungsbeispiels der Magnetlagervorrichtung 1 werden nur die Unterschiede zum ersten Ausführungsbeispiel aus Fig. 1 näher erläutert. Die Erläuterungen zum ersten Ausführungsbeispiel gelten in gleicher Weise oder sinngemäss auch für das zweite Ausführungsbeispiel. Gleiche Bezugsziffern bezeichnen die gleichen Merkmale, die unter Bezugnahme auf das erste Ausführungsbeispiel erläutert wurden, oder funktionell gleichwertige Merkmale.

Ein Unterschied dieses Ausführungsbeispiels einer Magnetlagervorrichtung 1 zu dem Ausführungsbeispiel aus Fig. 1 ist, dass der Rückschluss 28 anders ausgestaltet ist. Der Rückschluss 28 ist ringförmig mit einem metallischen Band 29 ausgestaltet, welches sich von einem radial inneren Anfang 291 zu einem radial äusseren Ende 292 erstreckt. Das Band 29 formt mehrere Bandwicklungen 293, die bezüglich der radialen Richtung R flächig aneinander anliegen. Die Längsschenkel 26 werden am ersten Ende 261 durch eine axiale Stirnfläche 265 begrenzt, an welchen der Rückschluss 28 anliegt. Der Rückschluss 28 formt einen Kreisring, dessen radiale Breite gleich der radialen Breite der Stirnflächen 265 der Längsschenkel 26 ist. Das heisst, der radial innere Anfang 291 ist in axialer Richtung A bündig mit einer radial innenliegenden Innenfläche 266 des Längsschenkels 26 und das radial äussere Ende 292 ist in axialer Richtung A bündig mit einer radial aussenliegenden Aussenfläche 267 des Längsschenkels 26.

Dies hat unter anderem den Vorteil, dass im Inneren des Stators 2 mehr Platz vorhanden ist, der genutzt werden kann, um andere Komponenten darin zu verbauen. Dadurch kann der Stator 2 samt Statorgehäuse 21 kleiner und kompakter gebaut werden, wodurch die Einsatzflexibilität der Magnetlagervorrichtung 1 erhöht wird.

Ein weiterer Vorteil bei solch einer Anordnung des Rückschlusses 28 ergibt sich aus der Anordnung der Bandwicklungen 293 des Bands 29 des Ringbandkerns. Dadurch dass die Bandwicklungen 293 senkrecht auf der radialen Richtung R angeordnet sind, weisen sie eine Orientierung auf, die parallel zum Magnetfeldverlauf in den Längsschenkeln 26 ist. Dadurch tritt das Magnetfeld aus den Längsschenkeln 26 in axialer Richtung A in den Rückschluss 28 ein und somit parallel zu den Bandwicklungen 293. Das heisst, das Magnetfeld durchdringt keine der Bandwicklungen 293 in radialer Richtung R wodurch Wirbelstromverluste vermieden werden.

Ein weiterer Unterschied zum Ausführungsbeispiel in Fig. 1 ist, dass die Erstreckung der Schlitze 254 in den Längsschenkel 26 länger ist. Dies kann je nach Einsatzgebiet der Magnetlagervorrichtung 1 von Vorteil sein, um eine höhere Reduktion der Wirbelstromverluste erreichen zu können.

Des Weiteren unterscheidet sich der Rotor 3 in diesem Ausführungsbeispiel von dem im Ausführungsbeispiel in Fig. 1. Der hiervorliegende Rotor 3 ist als sogenannter Vier-Polpaariger Rotor ausgestaltet.

Es ist aber natürlich auch möglich, die in diesem Ausführungsbeispiel gezeigte Magnetlagervorrichtung 1 mit jeglichen anderen Rotoren 3 zu betreiben. Einige davon wurden in vorherigen Abschnitten bereits erläutert.

Es versteht sich von selbst, dass alle in der Figurenbeschreibung gezeigten Ausführungsbeispiele mit ihren jeweiligen Eigenarten untereinander in jeglicher Form kombinierbar sind.

Des Weiteren ist es möglich, dass sämtliche gezeigten Ausführungsbeispiele eines Spulenkerns 25 derart ausgestaltet sein können, dass der dem Rotor 3 zu Verfügung stehende Platz in der Magnetlagervorrichtung 1 vergrössert wird. Dies gelingt durch eine spezielle äussere Form der Spulenkerne 25.

Dabei wird der Spulenkern 25 in einen axial unteren Abschnitt und einen axial oberen Abschnitt aufgeteilt, wobei der untere Abschnitt und der obere Abschnitt bezüglich der axialen Richtung A benachbart zueinander angeordnet sind. Der Querschenkel 27 ist an dem axial oberen Abschnitt angeordnet. Für jeden Spulenkern 25 weist die Stirnfläche 272 des Querschenkels 27 von dem axial unteren Abschnitt des zugehörigen Längsschenkels 26 einen ersten Abstand in radialer Richtung auf, und von dem axial oberen Abschnitt einen zweiten Abstand in radialer Richtung, wobei der zweite Abstand grösser ist als der erste Abstand. Dies bedeutet, dass jeder Längsschenkel 26 derart ausgestaltet ist, dass der axial obere Abschnitt bezüglich des axial unteren Abschnitts in radialer Richtung nach aussen verschoben ist, sodass sich der für den Rotor 3 zur Verfügung stehende Platz zwischen den Stirnflächen 272 vergrössert, ohne dass dabei die Gefahr eines direkten Übertritts des magnetischen Flusses zwischen dem Längsschenkel 26 und dem magnetisch wirksamen Kern 31 des Rotors 3 besteht. Dadurch, dass die axial oberen Abschnitte bezüglich der radialen Richtung und relativ zu den axial unteren Abschnitten radial nach aussen versetzt sind, vergrössert sich der Abstand, nämlich der zweite Abstand, zwischen den Längsschenkeln 26 und den Stirnflächen 272 im Bereich der axial oberen Abschnitte. Dadurch vergrössert sich auch der Abstand zwischen dem magnetisch wirksamen Kern 31 des Rotors und den Längsschenkeln 26 insbesondere im Bereich der axial oberen Abschnitte.

Solche gerade beschriebenen Spulenkerne 25 sind analog zu denen in Fig. 3 in der Europäischen Patentanmeldung EP4084304A1 abgebildeten Spulenkerne ausgebildet.

Fig. 8 zeigt eine schematische Schnittdarstellung für eine Ausgestaltung eines Statorgehäuses 21. In den Ausführungsbeispielen in Fig. 1 und Fig. 7 ist dieses Statorgehäuse 21 aus Gründen der besseren Übersicht nicht dargestellt. Fig. 8 soll lediglich der Anschauung dienen, um zu zeigen, wie eine für den Betrieb der Magnetlagervorrichtung 1 notwendige Umkapselung des Inneren des Stators 2 aussieht. Aus diesem Grund sind die anderen Komponenten des Stators 2 nur schematisch dargestellt und rein anschaulich zu verstehen.

Es sind ebenso Ausgestaltungen des Statorgehäuses 21 möglich, bei denen die becherförmige Ausnehmung 211 in eine Bohrung übergeht, die sich zentral entlang der Mittelachse des Stators 2 in axialer Richtung A durch das gesamte Statorgehäuse 21 erstreckt.

Beim Betrieb der Magnetlagervorrichtung 1 in Bereichen in denen z.B. chemisch aggressive Substanzen zum Einsatz kommen, ist es wichtig, dass das Innere des Stators 2 sicher umkapselt ist und somit vor diesen Substanzen geschützt wird. Damit trotzdem ein Rotor 3 verwendet werden kann, weist das Statorgehäuse 21 eine becherförmige Ausnehmung 211 auf, in welche der Rotor 3 eingesetzt werden kann.

## Patentansprüche

1. Magnetlagervorrichtung zur berührungslos magnetischen Lagerung eines Rotors (3), der einen scheibenförmigen oder ringförmigen magnetisch wirksamen Kern (31) umfasst, wobei die Magnetlagervorrichtung einen Stator (2) aufweist, welcher eine Mehrzahl von Spulenkernen (25) umfasst, wobei jeder Spulenkern (25) geblecht aus Elementen (253) hergestellt ist, wobei die Elemente (253) in Umfangsrichtung des Stators (2) gestapelt sind, wobei jeder Spulenkern (25) eine erste laterale Begrenzungsfläche (255) und eine zweite laterale Begrenzungsfläche (256) aufweist, wobei jeder Spulenkern (25) einen Längsschenkel (26) umfasst, welcher sich von einem ersten Ende (261) in einer axialen Richtung (A) bis zu einem zweiten Ende (262) erstreckt, sowie einen Querschenkel (27), welcher an dem zweiten Ende (262) des Längsschenkels angeordnet ist, und sich in einer radialen Richtung erstreckt, die senkrecht zur axialen Richtung (A) ist, wobei an jedem Längsschenkel (26) mindestens eine konzentrierte Wicklung (61) vorgesehen ist, welche den jeweiligen Längsschenkel (26) umgibt, wobei der Stator (2) ferner eine becherförmige Ausnehmung (211) aufweist, in welche der Rotor (3) einsetzbar ist, wobei die becherförmige Ausnehmung (211) an einem axialen Ende des Stators (2) angeordnet ist, und wobei die Querschenkel (27) um die becherförmige Ausnehmung (211) herum angeordnet sind, **dadurch gekennzeichnet, dass**
mindestens eine der ersten oder der zweiten lateralen Begrenzungsflächen (255, 256) mindestens einen Schlitz (254) aufweist.

2. Magnetlagervorrichtung nach Anspruch 1, wobei die Elemente (253) aus Elektroblech hergestellt sind.

3. Magnetlagervorrichtung nach einem der vorangehenden Ansprüche, wobei sich der mindestens eine Schlitz (254) nur im Querschenkel (27) erstreckt.

4. Magnetlagervorrichtung nach einem der vorangehenden Ansprüche, wobei sich der mindestens eine Schlitz (254) im Querschenkel (27) und im Längsschenkel (26) erstreckt.

5. Magnetlagervorrichtung nach Anspruch 4, wobei der mindestens eine Schlitz (254) eine Rundung (2541) aufweist, welche den Schlitz (254) aus der radialen Richtung (R) in die axiale Richtung (A) umlenkt.

6. Magnetlagervorrichtung nach einem der vorangehenden Ansprüche, wobei sich der mindestens eine Schlitz (254) von der ersten lateralen Begrenzungsfläche (255) bis zu der zweiten lateralen Begrenzungsfläche (256) erstreckt.

7. Magnetlagervorrichtung nach einem der vorangehenden Ansprüche, wobei die Erstreckung des mindestens einen Schlitzes (254) in Umfangsrichtung des Stators (2) gesehen kürzer ist als der Abstand der ersten lateralen Begrenzungsfläche von der zweiten lateralen Begrenzungsfläche.

8. Magnetlagervorrichtung nach einem der vorangehenden Ansprüche, wobei mehrere Schlitze (254) vorgesehen sind, welche parallel oder zumindest näherungsweise parallel zueinander angeordnet sind.

9. Magnetlagervorrichtung nach einem der vorangehenden Ansprüche, wobei der Spulenkern (25) an einem axial oberen Ende (252) eine Abrundung (257) aufweist, welche den Spulenkern aus der axialen Richtung (A) in die radiale Richtung (R) umlenkt.

10. Magnetlagervorrichtung nach einem der vorangehenden Ansprüche, wobei am ersten Ende (261) der Längsschenkel (26) ein Rückschluss (28) angeordnet ist, der die ersten Enden (261) aller Längsschenkel (26) verbindet, wobei der Rückschluss (28) ringförmig mit einem metallischen Band (29) ausgestaltet ist, welches sich von einem radial inneren Anfang (291) zu einem radial äusseren Ende (292) erstreckt, wobei das Band (29) mehrere Bandwicklungen (293) formt, die bezüglich der radialen Richtung (R) flächig aneinander anliegen.

11. Magnetlagervorrichtung nach einem der vorangehenden Ansprüche, wobei an jedem Längsschenkel (26) zwei konzentrierte Wicklungen (61a, 61b) vorgesehen sind, von denen jede den jeweiligen Längsschenkel (26) umgibt, und die bezüglich der axialen Richtung (A) benachbart zueinander angeordnet sind.

12. Magnetlagervorrichtung, nach einem der vorangehenden Ansprüche, wobei sowohl in der ersten lateralen Begrenzungsfläche als auch in der zweiten lateralen Begrenzungsfläche jeweils mindestens ein Schlitz vorgesehen ist.

13. Magnetlagervorrichtung nach einem der vorangehenden Ansprüche, wobei der Stator (2) zur Erzeugung eines Drehmoments ausgestaltet ist, mit welchem der Rotor (3) berührungslos magnetisch zur Rotation um die axiale Richtung (A) antreibbar ist.

14. Elektromagnetischer Drehantrieb, der als Tempelmotor ausgestaltet ist, **dadurch gekennzeichnet, dass** der elektromagnetische Drehantrieb eine Magnetlagervorrichtung (1) gemäss Anspruch 13 umfasst, sowie einen Rotor (3) mit einem scheibenförmigen oder ringförmigen magnetisch wirksamen Kern (31), wobei der Rotor (3) in die becherförmige Ausnehmung (211) einsetzbar ist, und wobei der Rotor (3) als Rotor (3) des elektromagnetischen Drehantriebs ausgestaltet ist.

## Claims

1. Magnetic levitation device for contactless magnetic levitation of a rotor (3), which comprises a disk-shaped or ring-shaped magnetically effective core (31), wherein the magnetic levitation device has a stator (2), which comprises a plurality of coil cores (25), wherein each coil core (25) is made of elements (253) in sheet metal, wherein the elements (253) are stacked in the circumferential direction of the stator (2), wherein each coil core (25) has a first lateral boundary surface (255) and a second lateral boundary surface (256), wherein each coil core (25) comprises a longitudinal leg (26), which extends from a first end (261) in an axial direction (A) to a second end (262), as well as a transverse leg (27), which is arranged at the second end (262) of the longitudinal leg, and which extends in a radial direction, which is perpendicular to the axial direction (A), wherein at least one concentrated winding (61) is provided on each longitudinal leg (26), which surrounds the respective longitudinal leg (26), wherein the stator (2) further has a cup-shaped recess (211) into which the rotor (3) can be inserted, wherein the cup-shaped recess (211) is arranged at an axial end of the stator (2), and wherein the transverse legs (27) are arranged around the cup-shaped recess (211), **characterized in that** at least one of the first or the second lateral boundary surfaces (255, 256) has at least one slot (254).

2. Magnetic levitation device according to claim 1, wherein the elements (253) are made of electrical sheet metal.

3. Magnetic levitation device according to any one of the preceding claims,
wherein the at least one slot (254) extends only in the transverse leg (27).

4. Magnetic levitation device according to any one of the preceding claims, wherein the at least one slot (254) extends in the transverse leg (27) and in the longitudinal leg (26).

5. Magnetic levitation device according to claim 4, wherein the at least one slot (254) has a rounding (2541) which redirects the slot (254) from the radial direction (R) to the axial direction (A).

6. Magnetic levitation device according to any one of the preceding claims, wherein the at least one slot (254) extends from the first lateral boundary surface (255) to the second lateral boundary surface (256).

7. Magnetic levitation device according to any one of the preceding claims, wherein the extension of the at least one slot (254) is shorter than the distance of the first lateral boundary surface from the second lateral boundary surface when viewed in the circumferential direction of the stator (2).

8. Magnetic levitation device according to any one of the preceding claims, wherein several slots (254) are provided which are arranged parallel or at least approximately parallel to each other.

9. Magnetic levitation device according to any one of the preceding claims, wherein the coil core (25) has a rounding off (257) at an axially upper end (252), which redirects the coil core from the axial direction (A) to the radial direction (R).

10. Magnetic levitation device according to any one of the preceding claims, wherein a back iron (28) is arranged at the first end (261) of the longitudinal legs (26), which connects the first ends (261) of all longitudinal legs (26), wherein the back iron (28) is designed in a ring-shaped manner with a metallic strip (29) which extends from a radially inner beginning (291) to a radially outer end (292), wherein the strip (29) forms several strip windings (293) which lie flat against one another with respect to the radial direction (R).

11. Magnetic levitation device according to any one of the preceding claims, wherein two concentrated windings (61a, 61b) are provided on each longitudinal leg (26), each of which surrounds the respective longitudinal leg (26), and which are arranged adjacent to one another with respect to the axial direction (A).

12. Magnetic levitation device according to any one of the preceding claims, wherein at least one slot is provided in each case both in the first lateral boundary surface and in the second lateral boundary surface.

13. Magnetic levitation device according to any one of the preceding claims, wherein the stator (2) is designed to generate a torque with which the rotor (3) can be driven magnetically without contact for rotation about the axial direction (A).

14. Electromagnetic rotary drive, which is designed as a temple motor, **characterized in that** the electromagnetic rotary drive comprises a magnetic levitation device (1) according to claim 13, as well as a rotor (3) with a disk-shaped or ring-shaped magnetically effective core (31), wherein the rotor (3) can be inserted into the cup-shaped recess (211), wherein the rotor (3) is designed as the rotor (3) of the electromagnetic rotary drive.

## Revendications

1. Dispositif à lévitation magnétique pour la lévitation magnétique sans contact d'un rotor (3), qui comprend un noyau (31) magnétiquement actif en forme de disque ou en forme d'anneau, dans lequel le dispositif à lévitation magnétique présente un stator (2), qui comprend une pluralité de noyaux de bobine (25), dans lequel chaque noyau de bobine (25) est constitué d'éléments (253) en tôle, dans lequel les éléments (253) sont empilés dans la direction circonférentielle du stator (2), dans lequel chaque noyau de bobine (25) présente une première surface de délimitation latérale (255) et une deuxième surface de délimitation latérale (256), dans lequel chaque noyau de bobine (25) comprend une branche longitudinale (26), qui s'étend d'une première extrémité (261) dans une direction axiale (A) à une deuxième extrémité (262), ainsi qu'une branche transversale (27), qui est agencée au niveau de la deuxième extrémité (262) de la branche longitudinale, et qui s'étend dans une direction radiale, qui est perpendiculaire à la direction axiale (A), dans lequel au moins un enroulement concentré (61) est prévu sur chaque branche longitudinale (26), qui entoure la branche longitudinale respective (26), dans lequel le stator (2) présente en outre un évidement en forme de godet (211), dans lequel le rotor (3) peut être inséré, dans lequel l'évidement en forme de godet (211) est agencé au niveau d'une extrémité axiale du stator (2), et dans lequel les branches transversales (27) sont agencées autour de l'évidement en forme de godet (211), **caractérisé en ce qu'**au moins l'une parmi la première ou la deuxième surface de délimitation latérale (255, 256) présente au moins une fente (254).

2. Dispositif à lévitation magnétique selon la revendication 1, dans lequel les éléments (253) sont constitués de tôle électrique.

3. Dispositif à lévitation magnétique selon l'une quelconque des revendications précédentes, dans lequel l'au moins une fente (254) s'étend uniquement dans la branche transversale (27).

4. Dispositif à lévitation magnétique selon l'une quelconque des revendications précédentes, dans lequel l'au moins une fente (254) s'étend dans la branche transversale (27) et dans la branche longitudinale (26).

5. Dispositif à lévitation magnétique selon la revendication 4, dans lequel l'au moins une fente (254) présente un arrondi (2541), qui dévie la fente (254) de la direction radiale (R) à la direction axiale (A).

6. Dispositif à lévitation magnétique selon l'une quelconque des revendications précédentes, dans lequel l'au moins une fente (254) s'étend de la première surface de délimitation latérale (255) à la deuxième surface de délimitation latérale (256).

7. Dispositif à lévitation magnétique selon l'une quelconque des revendications précédentes, dans lequel l'extension de l'au moins une fente (254) est plus courte que la distance de la première surface de délimitation latérale à la deuxième surface de délimitation latérale, vue dans la direction circonférentielle du stator (2).

8. Dispositif à lévitation magnétique selon l'une quelconque des revendications précédentes, dans lequel plusieurs fentes (254) sont prévues, qui sont disposées parallèlement ou au moins approximativement parallèlement les unes aux autres.

9. Dispositif à lévitation magnétique selon l'une quelconque des revendications précédentes, dans lequel le noyau de bobine (25) présente à une extrémité axialement supérieure (252) un arrondissement (257), qui dévie le noyau de bobine de la direction axiale (A) à la direction radiale (R).

10. Dispositif à lévitation magnétique selon l'une quelconque des revendications précédentes, dans lequel un fer de retour (28) est disposé à la première extrémité (261) des branches longitudinales (26), lequel relie les premières extrémités (261) de toutes les branches longitudinales (26), dans lequel le fer de retour (28) est conçu de manière annulaire avec une bande métallique (29), qui s'étend d'un début radialement intérieur (291) à une extrémité radialement extérieure (292), dans lequel la bande (29) forme plusieurs enroulements de bande (293), qui reposent à plat les uns contre les autres par rapport à la direction radiale (R).

11. Dispositif à lévitation magnétique selon l'une quelconque des revendications précédentes, dans lequel deux enroulements concentrés (61a, 61b) sont prévus sur chaque branche longitudinale (26), lesquels entourent à chaque fois la branche longitudinale respective (26) et sont disposés de manière adjacente les uns aux autres par rapport à la direction axiale (A).

12. Dispositif à lévitation magnétique selon l'une quelconque des revendications précédentes, dans lequel au moins une fente est prévue à chaque fois à la fois dans la première surface de délimitation latérale et dans la deuxième surface de délimitation latérale.

13. Dispositif à lévitation magnétique selon l'une quelconque des revendications précédentes, dans lequel le stator (2) est conçu pour générer un couple, avec lequel le rotor (3) peut être entraîné magnétiquement sans contact pour tourner autour de la direction axiale (A).

14. Entraînement rotatif électromagnétique, qui est conçu sous forme de moteur de temple, **caractérisé en ce que** l'entraînement rotatif électromagnétique comprend un dispositif à lévitation magnétique (1) selon la revendication 13, ainsi qu'un rotor (3) avec un noyau magnétiquement actif en forme de disque ou en forme d'anneau (31), dans lequel le rotor (3) peut être inséré dans l'évidement en forme de godet (211), dans lequel le rotor (3) est conçu sous forme de rotor (3) de l'entraînement rotatif électromagnétique.
